# EUROPÄISCHE PATENTSCHRIFT

(11) **EP 0 864 063 B1**
(45) Veröffentlichungstag und Bekanntmachung des Hinweises auf die Patenterteilung: **26.02.2003**
(21) Anmeldenummer: 96941041.4
(22) Anmeldetag: 27.11.1996
(51) Int. Cl.: F16M 11/04, G02B 7/00

(54) **Mikroskopstativ, insbesondere für ein Operationsmikroskop**
Microscope support, intended in particular for a surgical microscope
Pied pour un microscope, notamment pour un microscope d'intervention chirurgicale

(30) Priorität: 27.11.1995 CH 346795
(43) Veröffentlichungstag der Anmeldung: 16.09.1998
(73) Patentinhaber: Leica AG, 9435 Heerbrugg (CH)
(72) Erfinder: METELSKI, Andreas, CH-8590 Romanshorn (CH); WÄGER, Karl-Heinz, A-6840 Götzis (AT)
(74) Vertreter: Stamer, Harald, Dipl.-Phys.
(86) Internationale Anmeldenummer: EP9605241
(87) Internationale Veröffentlichungsnummer: WO97020166

(56) Entgegenhaltungen:
- EP-A- 0 476 551
- EP-A- 0 476 552
- EP-A- 0 554 711
- EP-A- 0 628 290
- DE-A- 3 313 155
- DE-C- 4 214 858
- FR-A- 2 645 070

## Beschreibung

In der Chirurgie finden mehr und mehr Operationsmikroskope Anwendung, die infolge ihres hohen Eigengewichtes von Stativen getragen werden müssen. Eine Reihe namhafter Hersteller brachte Stative auf den Markt, die in mechanischer und statischer Hinsicht den Anforderungen des Aufnehmens der Last des Operationsmikroskopes gut entsprechen. Die Anmelderin vertreibt z.B. Stative mit der Bezeichnung OH, die u.a. von Mitaka hergestellt wurden. Ein Beispiel für ein solches Stativ findet sich in der EP-A-628290. Zeiss veröffentlichte ein Stativ z.B. in der EP-476552. Die meisten der moderneren Stative verfügen über Parallelogrammträger, um die Last der Operationsmikroskope über möglichst grosse Distanzen biege- und verwindungsfrei tragen zu können, so dass die Bewegungsfreiheit und der Aktionsradius der Mikroskope möglichst gross sind.

In der EP-A-628290 ist beispielsweise ein solcher Aufbau dargestellt. Der dort z.B. in Fig.1 dargestellte Aufbau verfügt über einen C-förmigen massiven Grundständer, der einen ersten vertikal erstreckten Parallelogrammträger um eine Vertikalschwenkachse schwenkbar trägt. Der erste Parallelogrammträger trägt einen zweiten, horizontal erstreckten Parallelogrammträger, der um eine erste Horizontalschwenkachse schwenkbar ist. Durch eine Verbindung zwischen den beiden Parallelogrammträgern mit einem zusätzlichen Anlenkpunkt am Grundständer wird erreicht, dass die beiden vertikalen Teilarme des zweiten Parallelogrammträgers stets in einer vertikalen Lage sind.

Am peripheren vertikalen Teilarm ist über eine zweite Horizontalschwenkachse ein dritter, horizontal erstreckter Parallelogrammträger schwenkbar angebracht, dessen peripherer vertikaler Teilarm ebenso stets vertikal gehalten ist und einstückig mit einem zentralen vertikalen Teilarm eines vierten, vertikal erstreckten Parallelogrammträgers verbunden ist.

An einem unteren horizontalen Teilarm des vierten Parallelogrammträgers ist ein Operationsmikroskop dreh- bzw. schwenkbar befestigt, das somit über beliebige Freiheitsgrade verfügt, um durch einen Anwender in eine gewünschte Position gebracht und in dieser Position gehalten zu werden. Alle Parallelogrammträger verfügen über einen relativ massiven Hauptträger und einen schwächeren Nebenträger, der im wesentlichen ausschliesslich Züge oder Drücke überträgt. Aus statischen Gründen ergibt es sich, dass alle massiven Hauptträger dem durch das Stativ umschriebenen Raum, der in der Regel durch einen Anwender ausgenutzt wird, zugewandt sind.

Die Hauptträger sind dabei durch Kröpfungen aus ihrer - statisch wirksamen - an sich geraden Längserstreckung gebogen. Dieses war im Stand der Technik deshalb erwünscht, da man damit versuchte, den Raum für den Anwender möglichst gross zu gestalten.

Einer der Gedanken herkömmlicher Operationsstativ-Hersteller geht in die Richtung, dass grössere Massivität der Bauteile und höhere Gewichte (Ausgleichsgewichte) gut für' die Stabilität des Stativs während seiner Anwendung sind.

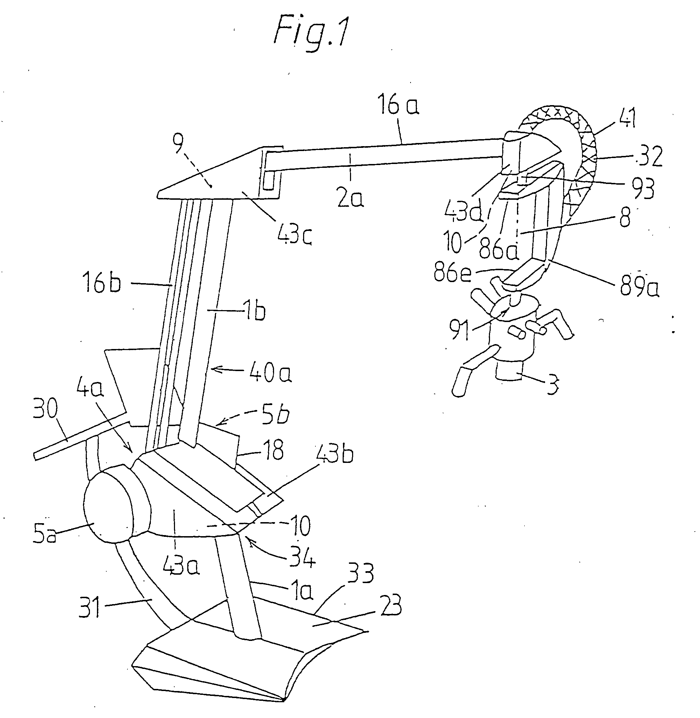

Auch die Firma Contraves brachte ein ähnliches Mikroskopstativ auf den Markt mit zwei getrennten Ausgleichsgewichten, wobei eines am ausgleichskraftübertragenden horizontalen Parallellenker in horizontaler Richtung und das andere an eben diesem in vertikaler Richtung verschiebbar ist. Ein solches Stativ ist beispielsweise auch in der älteren EP-B-476551 beschrieben. Bei diesem Stativ wurde auf die Kröpfung der Hauptträger verzichtet, was zu einer gewissen Einschränkung des Arbeitsraumes für den Anwender führte, wie man erkennen kann, wenn man z.B. Fig.1 der '551 Publikation mit Fig.4 der '290 Publikation vergleicht.

Ein Stativ entsprechend der jüngeren EP-A wurde durch die Anmelderin dieser EP-A (Mitaka) gemeinsam mit der Anmelderin auf den Markt gebracht. Zum Vorteil der Raumgewinnung gesellten sich jedoch auch bei diesem Stativ auch Nachteile:
Die Herstellung der gekröpften Arme ist wesentlich aufwendiger als die Anwendung gerader Arme. Durch die Kröpfung des Hauptträgers kommt es im Endbereich der parallelen Träger mit dem Nebenträger zu scherenartigen Überschneidungen, wie am besten den Fig.1-6 der EP-A entnommen werden kann. Diese Überschneidungen bergen vor allem für ungeübtes. Bedienpersonal einen gewissen Nachteil, da es bei Unachtsamkeit des Personals zum Einklemmen von Kabeln, Kleidungsstücken, Gegenständen oder sogar Extremitäten der Personen kommen könnte. Dies betrifft vor allem den Bereich in unmittelbarer Umgebung der Last, z.B. des Mikroskops, da dort ein Anwender in der Regel seine unmittelbaren Aktivitäten entfaltet.

Neu ging die Anmelderin bei der vorliegenden Erfindung auch von der erfindungsgemässen These aus, dass auch leichte Mikroskopstative eine gute Stabilität haben können, sofern sie konstruktiv über andere Bauteile verfügten. Als wesentlicher Vorteil gegenüber den bekannten massiven Stativen würde sich daraus eine bessere Transportierbarkeit und damit auch eine universellere Anwendbarkeit ergeben (weniger Probleme mit der Tragfähigkeit des Untergrundes usw.). Andererseits sollte es möglich sein, bei gleichem Gewicht grössere Aktionsradien für die Anwender zu erzielen.

Diese Aufgaben, zusammen mit dem Wunsch nach einer Produktionsvereinfachung, lagen dieser Erfindung zugrunde. So sollten z.B. längere, gerade Arme (ohne Kröpfungen) die gekröpften, aber schweren und daher eher kurzen Arme ablösen, um ohne Kröpfung (produktionsaufwendig) denselben Freiraum für den Anwender zu erzielen.

Andererseits sollte sich durch die neue erfindungsgemässe Konstruktion die im Stand der Technik bereits ausreichende Bewegungsgeometrie nicht verschlechtern.

Die zur Anwendung gelangenden erfindungsgemässen Träger, die bei Bedarf auch nach wie vor im Rahmen von Parallelogrammträgern angewendet werden können, sollen möglichst gerade, einfache Bauteile sein.

In Erfüllung dieser Aufgaben schuf die Anmelderin ein Mikroskop-Stativ gemäß Anspruch 1, der gegenüber der nicht vorveröffentlichten WO-A- 9 713 997 (Art. 54 (3) EPÜ) abgegrenzt ist.

Ausgehend vom Konzept des Ersatzes herkömmlicher paralleler Arme durch erfindungsgemässe faserverstärkte Kunststoffelemente, insbesondere Rohre, lässt sich Gewicht einsparen bei gleichzeitiger Erhöhung der Festigkeit oder der Aktionsradien. Das Stativ wird daher leichter. Dieser Effekt erhöht sich noch dadurch spürbar - da das Eigengewicht der Träger ebenso wie das Gewicht der Last durch Ausgleichsgewichte kompensiert werden muss - indem das Gewicht der Träger sich bei Reduktion des Gewichtes der Arme naturgemäss auch weiter reduzieren lässt.

Ein erfindungsgemässes Stativ wird somit transportfähiger und erfüllt auch die übrigen eingangs gestellten Aufgaben.

Zwar ist aus der DE-A-3 313 155 eine Messmaschine mit einem vertikalen und einem horizontalen Träger bekannt geworden, von denen wenigstens einer aus faserverstärkten Verbundstoffen aufgebaut ist. Der Schaffung dieser DE-Alag jedoch die Aufgabe zugrunde, bei reduziertem Gewicht eine höhere Genauigkeit (Temperaturunabhängigkeit für Messzwecke) und eine längere Lebensdauer zu finden (Seite 3 letzter Absatz und Seite 4 erster Absatz). Diese Aufgabe ist im Mikroskopstativbau unbekannt, da die Lebensdauer eines Stativs eher durch technologische Weiterentwicklungen als durch Abnützung überholt wird, und da allfällige Dimensionsänderungen durch Temperaturschwankungen die Benützbarkeit des Stativs nicht beeinträchtigen.

Abgesehen davon ist der Bau von Messmaschinen ein vom Mikroskopstativbau entferntes Fachgebiet. Dies gilt insbesondere auch für andere Fachgebiete, wie Fahrzeu-, Behälter- und Brückenbau.

Es lag somit nicht nahe, die Technologie gemäss der DE-A anzuwenden.

Bevorzugte Ausführungsformen der Erfindung setzen als Kunststoff Thermoplaste, Duroplaste, Thermoset (Epoxiharze) oder eine Mischung daraus ein, wobei als Fasermaterial Carbonfasern, Aramidfasern, Glas- oder Mineralfasern, Polyamidfasern oder eine Mischung daraus bevorzugt werden.

In diesem Zusammenhang wird ausdrücklich auf die frühere Schweizer Patentanmeldung der Anmelderin CH-02976/95 vom 12.10.1995 verwiesen, in der Aspekte dieser Erfindung bereits angeführt sind. Der Inhalt dieser Anmeldung ist durch die Prioritätsnachfolgeanmeldung. PCT/EP 96/04454 veröffentlicht worden. Die CH- bezieht sich auf ein Mikroskopstativ mit herkömmlichem Trägermaterial. Beide beschreiben ein völlig neues Mikroskop mit mehreren Erfindungen und vorteilhaften Varianten. Zum Zwecke einer späteren prioritätsbegünstigten Zusammenziehung der Lehren dieser zwei Anmeldungen ist daher ausdrücklich auf diese verwiesen und gilt deren Inhalt als hierin geoffenbart.

Gemäss einer Weiterbildung der Erfindung können mittels gezielter Einstellung der Faserorientierung die mechanischen und dynamischen Eigenschaften der Träger beeinflusst werden. So kann beispielsweise auf verschiedene Wickelmethoden der Fasern bzw. Lagen der Träger zurückgegriffen werden: Filament Winding, Flechtschlauch, Gewebe und Gelege, unidirektional bzw. in speziellem Winkel gelegt bzw. orientiert. So ist z.B. bei einem konkreten Ausführungsbeispiel der senkrechte Hauptständer, der das Hauptlager des Stativs trägt, mit einem isotropen Gelege (Rohrrichtung = 0°, Gelegerichtung der Lagen ± ca. 45°oder mit stumpferen Winkeln z.B. 0, ± 45-55° gelegt, woraus sich eine besonders hohe Torsionssteifigkeit ergibt, die vorteilhaft ist, zumal im eingebremsten Zustand des Stativs am Hauptständer hohe Drehmomente auftreten können, z.B. horizontaler Schlag im Bereich der Lastaufhängung oder Aktivieren der Bremsen während einer Bewegung des Mikroskops. Bei bevorzugter Verwendung von Carbon-Fasern mit Thermoset wird der Hauptständer bevorzugt aus einem ca. 4mm dicken Rohr mit 10cm Durchmesser und der erwähnten Winkelrichtung über Kreuz gelegt, mit einem Fasergewicht von ca. 130-160g/m². Als besonders bevorzugt kann die Winkelrichtung auch leicht variieren, so dass sich auch im wesentlichen gleich orientierte Lagen sperren: z.B. bei einer gewünschten Richtung von 55° wird eine Lage in Richtung 50° gelegt und gleich eine zweite mit einer Richtung von 60°, so dass sich im Schnitt die Richtung 55° ergibt. Dieses führt zu einer Verbesserung der Bruchfestigkeit des Trägers. Diese letztgenannte Art des Wickelns ist natürlich auch für die anderen Träger des Stativs von Vorteil, wenngleich für die übrigen Träger bevorzugt ein spitzerer Winkel bevorzugt ist, z.B. 0, ± (15-25°) bis (± 30-40°), da diese Rohre stärker auf Zug/Druck und Biegung beansprucht werden.

Die Erfindung bietet somit für die geometrisch erforderlichen Bauteile besonders geeignete und abgestimmte mechanische Bauteile mit geringerem Gewicht und höherer Festigkeit. Weitere spezielle Ausbildungen und Varianten dazu sind in den Patentansprüchen beschrieben.

Werden die Träger - insbesondere aus carbonfaserverstärktem Thermoset gefertigt, ergibt sich ein weiterer gegenüber dem Bekannten hervorragender Effekt, vor allem, wenn die Träger (Rohre) geschliffen, poliert und lackiert werden. Durch die Struktur der Carbonfasern, die unter dem Lack sichtbar wird, ergeben sich Farbeffekte und ein gefälliges, gegenüber bisherigem deutlich verbessertes Aussehen, das im Zuge der Auflockerung der Optik in den Operationssälen gewünscht und vorteilhaft ist.

Optisch besonders ansprechend ist dabei die Verwendung von ca. 10 Gewichtsteilen blauem Farblack im Verhältnis zu ca. 90 Gewichtsteilen Klarlack. Der Lack kann bevorzugt aufgespritzt oder im Tauchverfahren aufgebracht sein. Zur Vorbereitung werden gemäss eines Ausführungsbeispieles die mittels Aufblastechnologie, Spannfolienwickeltechnologie oder Autoklavtechnologie hergestellten carbonfaserverstärkten Rohre nach deren Aushärtung leicht angeschliffen, mit einer Schleifkörnung von ca. 300-400 (z.B. gemäss DIN 69100). Im Anschluss daran wird mit einem Poliermittel, z.B. M-Scotch-Brite 7448 (Handelsmarke der Firma 3M) nachpoliert, wobei bevorzugt die Schleif- und Polierrichtung in der jeweiligen Faserrichtung verlaufen sollte. Im Anschluss daran wird nach gründlicher Reinigung die erwähnte Lackmischung aufgespritzt und das Rohr fertig getrocknet. Um eine verbesserte Tiefenwirkung zu erhalten, kann eine zweite gleichartige Lackschicht im Nass-Nass-Verfahren aufgetragen werden. Den optisch ansprechenden Translutionseffekt kann man beeinflussen durch die Änderung der Farblackanteile: Farblackanteile mit mehr als 10 Gewichtsteilen bewirkt weniger Translution. Farblackanteile mit weniger als 10 Gewichtsteilen Farblack führt zu mehr Translution. Dieses kann bis zum "Glasklareffekt" erhöht werden. Beim ausgeführten Beispiel wurden mit gutem optischen Effekt 2-Komponenten-Epoxi-Lacke eingesetzt.

Die Verbindung zwischen den faserverstärkten Rohren bzw. Trägern und den übrigen Bauteilen erfolgt mittels einem metallischen Interface, das beispielsweise mittels Schrauben oder Splinten oder durch Klebung am jeweiligen Rohr bzw. Träger befestigt werden kann.

Eine weitere, vom obigen auch unabhängig einsetzbare Massnahme wird erfindungsgemäss gesetzt, um allfällige Vibrationen im mechanischen Aufbau zu dämpfen und derart eine verbesserte Betriebssicherheit zu erreichen:
Gemäss dieser besonderen Ausführung der Erfindung wird wenigstens eine Schnittstelle zwischen zwei tragenden Bauteilen des Stativs spannungsfrei gehalten. Dies kann im einfachsten Fall dadurch erfolgen, dass die Verbindung zwischen diesen Bauteilen (zB. eine Schraubverbindung) gelockert wird, so dass die Teile sich zwar nicht voneinander entfernen können, jedoch Vibrationen bzw. Schwingungen schlecht übertragen werden können.

Zusätzliche Dämpfungseffekte sind erzielbar, wenn an den entsprechenden Schnittstellen Dämpfungsmaterialien als Zwischenlager vorgesehen sind.

Als wesentlicher erfindungsgemässer Effekt wird durch diese Massnahme verhindert, dass am Mikroskop auftretende Vibrationen, ausgelöst durch kleine Stösse oder Positionsänderungen, nicht das gesamte Stativ durchlaufen und gegebenenfalls an der Aufstellfläche (z.B. Fussboden oder Decke) reflektiert, durch das Stativ an ihren Ursprung zurückgelangen.

Bevorzugte Stellen für die spannungsfreie Trennung sind jene Stellen am Stativ, an denen ausbalanciertes Gleichgewicht herrscht und daher kaum Biegespannungen auftreten. Bei einem Ausführungsbeispiel wurde als solche Stelle jene unmittelbar unter dem Hauptlager im Ständer vorgesehen, da das Stativ über dem Hauptlager in einem ausbalancierten Zustand ist, insbesondere wenn es entsprechend der Anmeldung CH-02976/95 aufgebaut ist.

Weitere Bereiche zur Spannungsfreimachung und/oder Einlage von Dämpfungsmaterialien sind gegebenenfalls auch zwischen den Lagerstellen für Räder, Aufstellfüssschen o.dgl. und den übrigen Bauteilen des Stativs.

Bei Bedarf können die erfindungsgemässen Aufbauten vorteilhaft auch leichter als bisherige Metallösungen mit motorischen Stellantrieben versehen werden, die bei Bedarf ein automatisches Verstellen der Trägerarmneigungen zueinander ermöglichen, etwa um das Stativ - ferngesteuert - in eine vorgewählte Position zu bringen. Selbstverständlich benötigt es für eine entsprechende Vollautomatisierung noch Positions- oder Wegsensoren, die als Referenz für die erforderlichen Antriebe dienen. Andererseits können, gemäss einer besonderen Ausgestaltung der Erfindung, solche Antriebe aber auch als Schrittmotore mit integrierten Sensoren ausgebildet sein, so dass Antrieb und Messung in einem Arbeitsgang ablaufen. In weiterer Folge sind dadurch vollständig selbsttätig positionierbare Stative realisierbar, die im Gegensatz zu bekannten nunmehr( z.B. gemäss EP-A-554711 der Firma Carl Zeiss) auf elegante, leichtgewichtige Trägerelemente zurückgreifen und dementsprechend leicht bauen.

Im Zuge einer automatischen Ausbalancierung ist es darüber hinaus besonders vorteilhaft, wenn die erfindungsgemäss vorgesehene Vibrationsdämpfung vorhanden ist, da dadurch Messungen der Gleichgewichtszustände unbeeinflusst sind und die Balancierung schneller erfolgen kann, wobei die Geschwindigkeit infolge reduzierter Eigenmasse und daher reduzierter Trägheit grundsätzlich vergrösserbar ist.

Bei der Anwendung erfindungsgemässer neuartiger faserverstärkter Träger in Rohrform bietet sich als weiterer Vorteil an, elektrische Leitungen und/oder Flüssigkeits- oder Gasführungen ins Innere der Rohre zu verlegen, woraus sich platzsparende Effekte und eine integrierte Bauweise ergeben, die zudem nach aussen besser sterilisierbar sind.

Gemäss einer Weiterentwicklung sind Abdeckungen für Gelenke und exponierte Stellen des Stativs vorgesehen, die aus PU-Integralschaum, wie in der Anmeldung CH-02976/95 erwähnt. oder alternativ auch aus ABS Kunststoff - gegebenenfalls geschäumt - aufgebaut sein können. Diese Abdeckungen ergeben zusammen mit den neuartigen Trägermaterialien ein anwenderfreundliches Äusseres, das bei Berührung durch Bedienpersonal vorteilhafterweise sich nicht metallisch kalt anfühlt und zudem in den abgedeckten Bereichen noch stossgeschützt ist.

### Figurenbeschreibung

Die Figuren werden zusammenhängend beschrieben. Die Figurenbeschreibung und die Bezugszeichenliste bilden eine Einheit, die sich durch die übrigen Teile der Beschreibung und Ansprüche im Sinne einer vollständigen Offenbarung gegenseitig ergänzen. Gleiche Bezugszeichen bedeuten gleiche Bauteile. Gleiche Bezugszeichen mit unterschiedlichen Indizes bedeuten ähnliche, funktionsgleiche Bauteile. Die Figuren sind nur beispielhaft und nicht zwingend proportional richtig dargestellt. Die Figurenliste bildet mit der Figurenliste der erwähnten CH-02976/95 eine Einheit und ist im Falle der Kombination der Merkmale der drei Anmeldungen so wie die dazugehörende Beschreibung miteinander zu lesen.
Fig.1 zeigt eine Designansicht eines erfindungsgemässen Stativs mit erfindungsgemässen faserverstärkten Trägern und einem ebensolchen Ständer,
Fig.2 eine Symboldarstellung eines anderen neuartigen Stativs mit einer Zone spannungsfreier Trennung und
Fig.3 eine Symboldarstellung eines erfindungsgemäss angewendeten Stativträgerrohres mit der Faserorientierung, ein Detail eines Aufbaus nach Fig.1 im Schnitt.

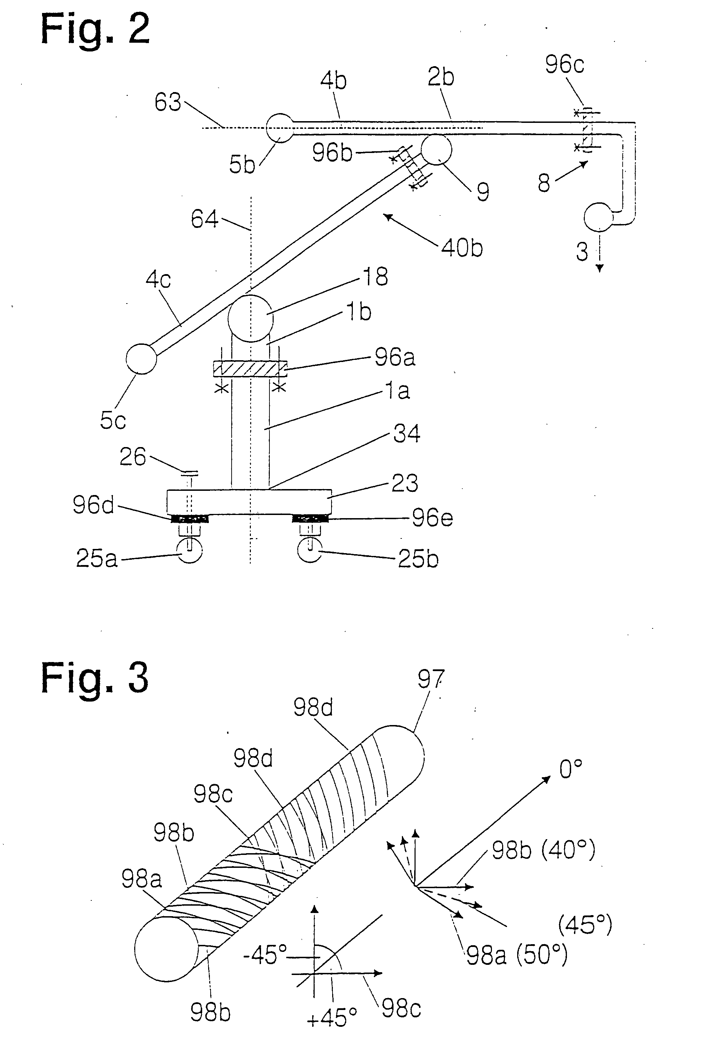

Ein Stativfuss 23 trägt einen Ständer 1a, der ein Hauptlager 18 aufnimmt. Gemäss dem Beispiel nach Fig. 2 ist der Ständer 1a zweigeteilt und mit einem Interface 96a versehen, das - hier im Beispiel - flanschartig ausgebildet ist und den Ständer in zwei Abschitte 1a und 1b teilt. Es könnte sich aber auch um einen am Ständer 1a befestigten Lagerbock für das Lager 18 handeln, so dass zwischen diesem und dem Ständer 1a das Interface ausgebildet ist. Wesentlich am Interface ist, dass es in vertikaler Richtung bzw. in Richtung der Ständererstreckung keine nennenswerte Spannung überträgt. Die dort symbolisch angedeuteten Schrauben sind z.B. nicht angezogen. Festigkeitstechnisch spielt dies wenig Rolle, da das gesamte Stativ über dem Lager 18 ohnedies ausbalanciert ist, so dass beim Interface 96a praktisch keine Biegekräfte auftreten.

Fig. 1 zeigt hingenen ein Beispiel, bei dem das Lager zwischen Ständer 1a und Ständer 1b angeordnet ist.

Im Rahmen der Erfindung können aber auch an anderen Stellen vergleichbare Interfaces eingerichtet sein 96b-e. Sie dienen stets dazu, Schwingungen im System möglichst nicht weiterzuleiten. Insbesondere in Bereichen mit Biegebeanspruchung, z.B. 96b,c können auch vibrationsdämpfende Zwischenlagen eingebaut sein, deren Aufgabe es ist, mechanische Schwingungen zu vernichten bzw. in Wärme umzuwandeln.

Die Träger 1,2,4,16,40 sind bevorzugt aus faserverstärktem Kunststoff aufgebaut und dementsprechend besonders leicht, so dass die Ausgleichsgewichte 5 ebenso leicht sein können und der Gesamtaufbau gegenüber herkömmlichen Aufbauten gewichtsreduziert ist.

Fig.3 zeigt symbolisch, wie die Fasern 98 im Beispiel orientiert sind. Vier Faserlagen schwanken in einem Winkel von ± 40°-50° zu 0° (Richtung des Rohres 97, das als Träger zum Einsatz kommt). Benachbarte Winkellagen (40°,50°) führen zu einer wirksamen Winkellage von 45° (98c), die für die Erzielung der Biege- bzw. Torsionssteifigkeit Bedeutung hat. Solche geringen Winkeldifferenzen erhöhen jedoch gegenüber einer einlagigen Winkelrichtung (z.B. nur 45°) etwas die Bruchfestigkeit, da sich die benachbarten Fasern offensichtlich gegenseitig die ansonsten bevorzugte Bruchrichtung entlang der Wickellage sperren.

Die erfindungsgemässe Brems (10)- und Messeinrichtung entsprechen beispielsweise der Detailzeichnung in Fig.18 und 19 der CH-02976/95, die Erfindung ist jedoch darauf nicht eingeschränkt.

Die Anwendung des neuen Stativs ist nicht auf die Mikroskopie eingeschränkt. Insbesondere der optische Bereich, Nah- und Fernvergrösserungen, aber auch Robotik o.dgl. fallen darunter.

### Weitere Bezugszeichenerläuterungen

Diese Bezugszeichenliste ist aus Gründen der Einfachheit und hinsichtlich einer möglichen späteren Kombination der Lehren der zwei Anmeldungen fortgeführt nach der Anmeldung CH-02976/95 und durch die neuen Bezugszeichen dieser Anmeldung ergänzt.
- 1a: Ständer, vorzugsweise am Boden rollbar; ist nur symbolisch mit geradem Stab dargestellt; könnte auch C-förmig, kastenförmig oder vergleichbar aufgebaut sein; muss nicht zwingend für eine Bodenmontage bzw. Aufstellung dienen, sondern könnte auch umgekehrt sein und an einer Decke, sonstigen Flächen oder Einrichtungsgegenständen - gegebenenfalls verfahrbar - montiert sein.
- 1b: Ständerkopf, ist ein Bauteil, der den Ständer nach oben hin zur Aufnahme der schwenkbaren Teile des Stativs abschliesst und insbesondere selbst drehbar am Ständer 1 sitzt.
- 2,a,b: Lastarm, eventuell aus mehreren Stäben aufgebaut; z.B. eine oder mehrere Parallelogrammführungen
- 3: Last, z.B. Mikroskop, könnte aber auch ein beliebiger Bauteil sein, der an einem Stativ zu halten ist, z.B. Roboterarm, Fernrohr o.dgl.
- 4,a,b,c: Ausgleichsarm, eventuell aus mehreren Stäben aufgebaut; z.B. eine oder mehrere Parallelogrammführungen
- 5a-c: Ausgleichsgewicht verschiebbar; kann einstückig oder insbesondere geteilt sein. Einer von verschiedenen Aspekten der Erfindung ist, dass zwei getrennte Ausgleichsgewichte für zwei von einander bewegungsgetrennte Ausgleichsfunktionen, nämlich um eine vertikale 64 und um eine horizontale Ebene 63 pendeln;
- 8: Lastaufhängung, umfasst Vorrichtungen zur Aufnahme eines Mikroskops oder sonstiger Lasten; insbesondere umfasst die Lastaufhängung gemäss einer Weiterbildung der Erfindung auch ein eigenes - dem Balanciersystem des Stativs selbst entsprechendes - Balanciersystem mit Last- und Ausgleichsarmen sowie Messeinrichtungen und Ausgleichsgewichten;
- 9: Schwenkachse (Horizontalschwenkachse) für den Lastarm 2 am und/oder Ausgleichsarm 4, an der diese aus einer horizontalen Ebene 63 schwenken können;
- 10: Bremseinrichtung, zur Abbremsung bzw. gegenseitigen Fixierung von zueinander beweglichen Bauteilen;
- 16: Zugarm horizontal (a) vertikal (b)
- 18: Drehpunkt bzw. Schnitt durch Drehachse bzw. Schwenkachse (Vertikalschwenkachse), um den das Stativ aus einer vertikalen Ebene schwenken kann.
- 23: Fuss des Stativs, dient zur Abstützung auf dem Boden, ist aber auch umgekehrt als Halteteil an einer Decke o.dgl. denkbar mit modifizierten Halteelementen (keine Rollen) ; 25a,b Räder für Fuss, können starr (nur eine bevorzugte Transportrichtung) oder drehbar befestigt sein; sind bevorzugt fixierbar oder durch parallele Aufstellfüsschen vom Boden abhebbar oder in den Fuss 23 einziehbar, um ein Absetzen des Fusses am Boden zu ermöglichen;
- 26: Stellmechanismus, z.B. Stellschraube
- 30: Transportgriff, zum Schieben oder Ziehen des Stativs; durch eine spezielle Griffstange 31 gibt er bevorzugt eine besondere Transportrichtung vor;
- 31: Griffstange
- 32: elektrische oder optische Versorgungsleitung o.dgl. für Funktionen des Stativs, z.B. Bremsen oder der Last (Mikroskop);
- 33: Gehäuse des Fusses 23, zur Tieferlegung des Gesamtschwerpunkts des Stativs aus Gussmaterial o.dgl. und/oder aus Kunststoff überzogen bzw. ausgebildet;
- 34: Drehlager
- 40a,b: Schwenkständer, ist der im Ruhezustand senkrechte Bauteil, der das Horizontalschwenklager 9 trägt bzw. dieses. in die Höhe hält; seine Funktion ist es, beim Schwenken aus' einer Vertikalebene 64 das Schwenklager 9 und damit den Lastarm 2 seitlich zu verschieben, so dass die Last 3 von der Vertikalebene 64 weg und auf sie zubewegt werden kann; er verfügt über eine vertikale Verlängerung unterhalb des Vertikalschwenklagers 18, die als Ausgleichsarm dient und das Ausgleichsgewicht 5b aufnimmt;
- 41: Schlauchkanal - insbesondere Wellschlauch - dient der Aufnahme und dem Schutz von elektrischen oder optischen Versorgungskabeln für die Last 3.
- 43a-d: Abdeckkappe, aus vorzugsweise allseitig geschlossenem Integralschaum, ist zu Servicezwecken leicht abnehmbar an den Gelenke aufweisenden Stellen des Stativs angebracht und verhindert im Falle von Zusammenstössen Verletzungen oder Beschädigungen; als weitere Vorteile sind das geringe Gewicht und die beliebige Formbarkeit herauszuheben, die dem Stativ mit geringen Mitteln auch ein gefälliges Aussehen verleihen;
- 86a-e: Träger horizontal
- 87a-c: Kette,. Riemen o.dgl.
- 89a: vertikaler Träger mit Ketten- bzw. Riementrieb; der Ketten- bzw. Riemen- oder Bandtrieb könnte im Rahmen der Erfindung auch mittels kardanischem Wellentrieb und entsprechenden Kegelzahnrädern in an sich bekannter Art und Weise ersetzt sein.
- 91: Endpunkt am unteren Horizontalträger, an dem die Last aufgehängt wird
- 93: Lager für Lastaufhängung 8
- 96a-e: Interface, Verbindung zwischen benachbarten Teilen des Stativs, gegebenenfalls mit elastischer, schwingungsdämpfender Zwischenlage z.B. aus einem Elastomerkautschuk mit hoher Bewegungsenergieumwandlung in Wärme
- 9: faserverstärktes Rohr für Stativträger
- 98: Faserlagen aus Carbon o.dgl.

## Patentansprüche

1. Mikroskopstativ, insbesondere für Operationsmikroskope, mit vertikalen und horizontalen Trägern (1,2,16,40), wobei wenigstens einer der Träger (1,2,16,40) aus faserverstärkten Verbundstoffen aufgebaut ist,
**dadurch gekennzeichnet, dass** wenigstens einer der aus faserverstärkten Verbundstoffen aufgebaute Träger (1,2,16,40) über ein metallisches Interface (96) verfügt, das ihn mit einem benachbarten Teil verbindet, oder das ihn zweiteilt und verbindet, wobei die Verbindung spannungsfrei gehalten ist und dass das Interface (96) eine dämpfende Zwischenlage, vorzugsweise aus einem elastomeren Material, umfasst.

2. Stativ nach Anspruch 1, **dadurch gekennzeichnet, dass** als Kunststoff Thermoplaste, Duroplaste, Thermoset (Epoxiharze) oder eine Mischung daraus vorgesehen sind, und/oder dass als Fasermaterial Carbon-, Aramid-, Glas-, Mineral- oder Polyamidfasern oder eine Mischung daraus vorgesehen sind.

3. Stativ nach Anspruch 1 oder 2, **dadurch gekennzeichnet, dass** die Fasern nach wenigstens einer der folgenden Wickelmethoden orientiert sind: Filament Winding, Flechtschlauch, Gewebe und Gelege, unidirektional oder in speziellem Winkel zur Trägererstreckung, wobei der Winkel als Funktion der Schwingungsdämpfung und/oder Steifigkeit bzw. Festigkeit gewählt ist.

4. Stativ nach einem der vorhergehenden Ansprüche, **dadurch gekennzeichnet, dass** die faserverstärkten Kunststoffe an ihrer Oberseite geschliffen, vorzugsweise poliert und/oder lackiert sind, wobei bevorzugt eine Lackmischung aus ca. 90 Gewichtsteilen Klarlack und ca. 10 Gewichtsteilen Farblack aufgetragen ist, wobei insbesondere Acryllacke, Epoxylacke, Epoxy-Acryllacke sowie bevorzugt Lacke auf Wasserbasis angewendet sind.

5. Stativ nach Anspruch 1, **dadurch gekennzeichnet, dass** das Interface (96a) unterhalb des Hauptschwenklagers (18) angeordnet ist, und/oder dass allfällige Räder (25) oder Stellfüssschen des Stativfusses gegenüber diesem mit einer Dämpfungsschicht beabstandet sind.

6. Stativ nach einem der vorhergehenden Ansprüche, **dadurch gekennzeichnet, dass** im Falle von gekreuzten Winkellagen der Faserrichtung der Fasern (98) je zwei Lagen zueinander einen geringen Winkel (z.B. ca.1°-29°) einschliessen, während wenigstens eine, vorzugsweise zwei weitere Lagen zu den beiden ersten Winkellagen einen grösseren Winkel (z.B. ca.30°-150°) einschliessen.

7. Stativ nach einem der vorhergehenden Ansprüche, **dadurch gekennzeichnet, dass** der den Ständer (1) bildende Träger folgende Faserorientierung aufweist: 0°(Rohrrichtung) ± (30°-60°), während die auf Biegung beanspruchten Träger (2,4,16,40) folgende Faserorientierung aufweisen: 0° (Rohrrichtung) ± (10°-30°)

## Claims

1. Microscope stand, particularly for surgical operation microscopes, with vertical and horizontal beams (1, 2, 16, 40), wherein at least one of the beams (1, 2, 16, 40) is constructed from fibre-reinforced composite materials, **characterised in that** at least one of the beams (1, 2, 16, 40) constructed from fibre-reinforced composite materials has a metallic interface (96), which connects it with an adjacent part, or which divides it in two and connects, wherein the connection is kept stress-free and that the interface (96) comprises a damping intermediate layer, preferably of an elastomeric material.

2. Stand according to claim 1, **characterised in that** thermoplastic material, duroplastic material, thermosetting material (epoxy resin) or a mixture thereof is provided as synthetic material and/or that carbon fibres, aramide fibres, glass fibres, mineral fibres or polyamide fibres or a mixture thereof are or is provided as fibre material.

3. Stand according to claim 1 or 2, **characterised in that** the fibres are oriented according to at least one of the following winding methods: filament winding, braided hose, woven fabric and non-woven fabric, unidirectionally or at a special angle to the beam length, wherein the angle is selected as a function of vibration damping and/or stiffness or strength.

4. Stand according to one of the preceding claims, **characterised in that** the fibre-reinforced synthetic materials are ground at their upper side, preferably polished and/or lacquered, wherein preferably a lacquer mixture of approximately 90 parts by weight of clear lacquer and approximately 10 parts by weight of coloured lacquer are applied, wherein, in particular, acrylic lacquer, epoxy lacquer, epoxy-acrylic lacquer as well as preferably lacquer on a water base are used.

5. Stand according to claim 1, **characterised in that** the interface (96a) is arranged below the main pivot bearing (18) and/or that, when applicable, wheels (25) or small adjusting feet of the stand base are spaced relative thereto by a damping layer.

6. Stand according to one of the preceding claims, **characterised in that** in the case of crossing angle layers the fibre direction of the fibres (98) of each two layers include a small angle (for example, about 1 to 29°) relative to one another, whilst at least one further layer, preferably two further layers, includes or include a greater angle (for example, approximately 30 to 150°) relative to the two first angle layers.

7. Stand according to one of the preceding claims, **characterised in that** the beam forming the upright (1) has the following fibre orientation: 0° (tube direction) ± (30 to 60°), whilst the beams (2, 4, 16, 40) loaded in bending have the following fibre orientation: 0° (tube direction) ± (10 to 30°).

## Revendications

1. Statif pour microscope, en particulier pour microscopes opératoires, avec des supports verticaux et horizontaux (1, 2, 16, 40), dans lequel au moins l'un des supports (1, 2, 16, 40) se compose de matières composites renforcées par fibres, **caractérisé en ce qu'**au moins l'un des supports (1, 2, 16, 40) composés de matières composites renforcées par fibres dispose d'une interface métallique (96) qui le relie à une partie adjacente ou le divise en deux et le relie, la liaison étant maintenue sans contrainte, et **en ce que** l'interface (96) comprend une couche intermédiaire amortissante, de préférence fabriquée dans un matériau élastomère.

2. Statif selon la revendication 1, **caractérisé en ce que** comme matière plastique, des matières thermoplastiques des matières duroplastiques, des matières thermodurcissables (résines époxy) ou un mélange de celles-ci sont prévues, et/ou que comme matière fibreuse, des fibres de carbone, des fibres d'aramide des fibres de verre, des fibres minérales ou des fibres de polyamide ou un mélange de celles-ci sont prévues.

3. Statif selon la revendication 1 or 2, **caractérisé en ce que** les fibres sont orientées selon au moins l'un des procédés d'enroulement suivants : enroulement de filaments, gaine tressée, tissé et non-tissé, de façon unidirectionnelle ou sous un angle spécial par rapport à l'extension du support, l'angle étant choisi en fonction de l'amortissement des oscillations et/ou de la rigidité ou de la résistance.

4. Statif selon l'une quelconque des revendications précédentes, **caractérisé en ce que** les matières plastiques renforcées par fibres sont abrasées à la surface, de préférence polies et/ou vernies, un mélange de vernis composé d'environ 90 pour-cent en poids de vernis clair et d'environ 10 pour-cent en poids de vernis coloré étant appliqué de préférence, des vernis acryliques, des vernis époxy, des vernis époxy-acryliques ainsi que de préférence des vernis à base d'eau étant utilisés en particulier.

5. Statif selon la revendication 1, **caractérisé en ce que** l'interface (96a) est agencée au-dessous du palier pivotant principal (18), et/ou **en ce que** d'éventuelles roulettes (25) ou pieds de réglage du pied de statif sont espacés par rapport à celui-ci par une couche d'amortissement.

6. Statif selon l'une quelconque des revendications précédentes, **caractérisé en ce que** dans le cas de positions relatives croisées de la direction de fibre des fibres (98), respectivement deux couches enferment mutuellement un petit angle (par exemple environ 1° à 29°), tandis qu'au moins une, de préférence deux autres couches enferment un plus grand angle (par exemple environ 30° à 150°) par rapport aux deux premières positions relatives.

7. Statif selon l'une quelconque des revendications précédentes, **caractérisé en ce que** le support constituant le statif (1) présente l'orientation de fibres suivante : 0° (direction de tube) ± (30° à 60°), tandis que les supports (2, 4, 16, 40) subissant une contrainte de flexion présentent l'orientation de fibres suivante : 0° (direction de tube) ± (10° à 30°).
